(19) Europäisches Patentamt
European Patent Office
Office européen des brevets

(11) Veröffentlichungsnummer : **0 149 427**
**B1**

(12) # EUROPÄISCHE PATENTSCHRIFT

(45) Veröffentlichungstag der Patentschrift : **08.07.87**

(21) Anmeldenummer : **84810620.9**

(22) Anmeldetag : **14.12.84**

(51) Int. Cl.⁴ : **C 07 C 93/14**, C 07 C121/34, C 07 C143/68, C 07 D317/28, C 07 D309/10, C 07 D319/12, C 07 D317/34, C 07 D493/08, A 01 N 33/10, A 01 N 37/34, A 01 N 37/02

(54) **(Poly-)Oxyalkylamino-diphenyläther mit herbizider Wirkung.**

(30) Priorität : **20.12.83 CH 6758/83**

(43) Veröffentlichungstag der Anmeldung : **24.07.85 Patentblatt 85/30**

(45) Bekanntmachung des Hinweises auf die Patenterteilung : **08.07.87 Patentblatt 87/28**

(84) Benannte Vertragsstaaten : **BE CH DE FR GB IT LI NL**

(56) Entgegenhaltungen :
**EP-A- 0 023 725**
**EP-A- 0 027 555**
**DE-C- 2 311 638**
**US-A- 4 419 124**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Dürr, Dieter, Dr.**
**Brändelistalweg 16**
**CH-4103 Bottmingen (CH)**

**EP 0 149 427 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue 3-(poly-)Oxyalkylamino-2'-chlor-4-nitro-4'-trifluormethyl-diphenyläther mit herbizider Wirkung, Verfahren zu deren Herstellung sie als Herbizide enthaltende Mittel sowie deren Verwendung als selektive Herbizide in Nutzpflanzenkulturen. Diese Verbindungen wirken ebenfalls gegen Insekten und Vertreter der Ordnung Akarina.

Die neuen 3-(poly-)Oxyalkylamino-2'-chlor-4-nitro-4'-trifluormethyl-diphenyläther entsprechen der Formel I

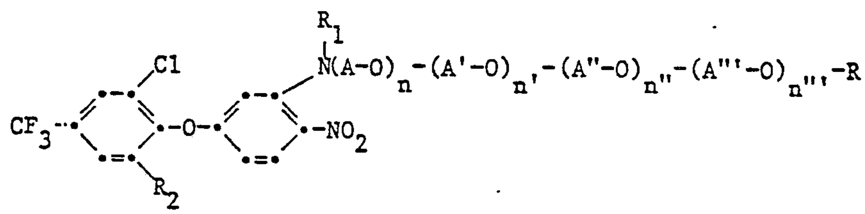 (I)

worin A, A', A" und A'" je ein gleicher oder verschiedender $C_1$-$C_4$-Alkylenrest der geradkettig oder verzweigt sein kann,

n eine Zahl 1 bis 5

n', n", n'" je Null oder eine Zahl von 1 bis 5 und

R ein $C_1$-$C_4$-Alkylrest, ein $C_1$-$C_4$-Alkylcarbonylrest, wobei die Alkylreste durch Halogen oder Cyan substituiert sein können ; ein Benzoyl- oder Phenylsulfonylrest der durch Halogen, $C_1$-$C_4$-Alkyl, $C_1$-$C_4$-Alkoxy, $C_1$-$C_4$-Halogenalkyl, $C_1$-$C_4$-Halogenalkoxy oder Nitro substituiert sein kann, oder falls A—O nicht Äethylenoxy und/oder falls die Summe von n, n', n" und n'" grösser als 1 ist, auch Wasserstoff bedeuten,

$R_1$ Wasserstoff, $C_1$-$C_4$-Alkyl, $C_1$-$C_4$-Hydroxyalkyl, $C_2$-$C_8$-Alkoxyalkyl, $C_1$-$C_4$-Alkylcarbonyl oder $C_1$-$C_4$-Halogenalkylcarbonyl, wobei die Reste R und $R_1$ über C—C oder C—O-Bindungen verzweigt oder, gegebenenfalls auch miteinander, ringgeschlossen sein können, und

$R_2$ Wasserstoff, Chlor oder Fluor bedeuten.

In dieser Formel umfassen die Alkylenreste A den Methylen-, Aethylen-, Propylen- und Butylenrest, wie die verzweigten Reste 1-Methyläthylen-, 2-Methyläthylen-, 1-Methylpropylen-, 2-Methylpropylen-, 3-Methylpropylen sowie 1,2 Dimethyläthylen. Bevorzugt sind die unverzweigten Aethylen-, Propylen- oder Methylenreste.

Die Alkylreste können verzweigt oder unverzweigt sein.

3-(subst.)Amino-2'-chlor-4-nitro-4'-trifluormethyldiphenyläther mit herbizider Wirkung sind bereits früher postuliert oder hergestellt worden, haben bisher aber keine praktische Bedeutung erlangt, siehe z. B. DE-A-2 311 638 oder US-A-4 046 798, DE-OS-2 304 006, DE-A-2 831 262 oder EP-A-27 555 ferner US-A-4 277 624. Die erfindungsgemässen Aminoalkyläther und deren Säureadditionssalze zeigen überraschenderweise bei guter herbizider Wirkung eine wertvolle Selektivität gegenüber Getreide, Reis, Mais, aber auch gegenüber dikotylen Kulturen, z. B. Soja. Sie scheinen in kleineren Aufwandmengen zu wirken als vergleichbare ähnliche bekannte Diphenyläther-Verbindungen.

Unter den Verbindungen der Formel I sind vor allen diejenigen wirksam, deren Alkylengruppen A, A', A", oder A'" durch Aethylen, 1-Methyläthylen und n-Propylen verkörpert sind. Beispiele für solche Verbindungen sind z. B.

3-(3,6-Dioxa-octylamino)-2'-chlor-4-nitro-4'-trifluormethyldiphenyläther,
3-(3-Oxa-heptylamino)-2'-chlor-4-nitro-4'-trifluormethyldiphenyläther,
2'-Chlor-3-(3,6,9-trioxa-decylamino)-4-nitro-4'-trifluormethyldiphenyläther,
2'-Chlor-4-nitro-3-(3,6,9-trioxa-nonylamino)-4'-trifluormethyldiphenyläther,
2'-Chlor-3-(4,7,10-trioxa-undec-2-ylamino)-4-nitro-4'-trifluormethyldiphenyläther,
2'-Chlor-3-(4,7-dioxa-oct-2-ylamino)-4-nitro-4'-trifluormethyldiphenyläther,
3-(3,6-Dioxa-decylamino)-2'-chlor-4-nitro-4'-trifluormethyldiphenyläther,
3-(4,7,10-Trioxa-dodec-2-ylamino)-2'-chlor-4-nitro-4'-trifluormethyldiphenyläther,
2'-Chlor-3-(4,7,10,13-tetraoxa-tetradec-2-ylamino)-4-nitro-4'-trifluormethyldiphenyläther,
3-(2,5,8-Trimethyl-3,6,9,12,15-pentaoxa-nonadecylamino)-2'-chlor-4-nitro-4'-trifluormethyldiphenyläther oder
2'-Chlor-3-(3-Oxa-butylamino)-4-nitro-4'-trifluormethyl-diphenyläther.

Die Verbindungen der Formel I werden nach verschiedenen, an sich bekannten Synthese-Wegen hergestellt.

Gemäss einem ersten Verfahren werden die 3-(poly-)Oxyalkylamino-2'-chlor-4-nitro-4'-trifluormethyldiphenyläther der Formel I hergestellt, indem man 2'-Chlor-3,4-dinitro-4'-trifluormethyldiphenyläther der Formel II

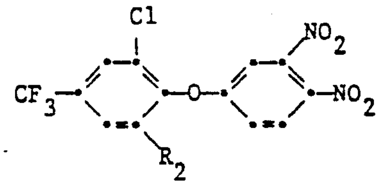 (II)

mit einem (poly-)Alkoxyamin der Formel III umsetzt.

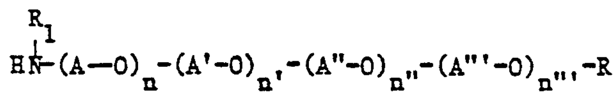 (III)

worin A, A', A'', A''', n, n', n'', n''', R und $R_1$ die unter Formel I gegebene Bedeutung haben.

Die Umsetzung erfolgt vorteilhafterweise in einem den Reaktionspartnern gegenüber inerten Lösungsmittel, gegebenenfalls in Anwesenheit der molaren Menge eines säurebindenden Mittels. Die Temperatur dieser Umsetzung kann zwischen der Raumtemperatur und dem Siedepunkt des Reaktionsgemisches variieren.

Gemäss einem zweiten Verfahren werden die 3-(poly-)Oxyalkylamino-2'-chlor-4-nitro-4'-trifluormethyldiphenyl-äther der Formel I auch hergestellt, indem man einen 3-(poly-)Alkoxyaminophenol der Formel IV

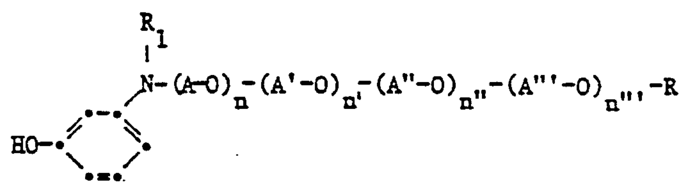 (IV)

worin A, A', A'', A''', n, n', n'', n''', R und $R_1$ die unter Formel I gegebene Bedeutung haben, zuerst mit einem 1,2-Dichlor-4-trifluormethylbenzol der Formel V kondensiert

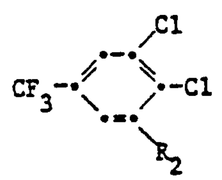 (V)

worin $R_2$ die unter Formel I gegebene Bedeutung hat und das Kondensationsprodukt der Formel VI

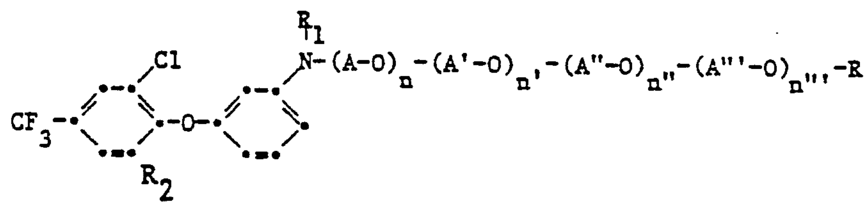 (VI)

worin A, A', A'', A''', n, n', n'', n''', R, $R_1$ und $R_2$ die unter Formel I gegebene Bedeutung haben, anschliessend mit Salpetersäure nitriert, wobei, falls $R_1$ Wasserstoff bedeutet, zuerst ein Acylrest $R_1'$ als Schutzgruppe eingeführt werden muss, der nach der Nitrierung durch Behandeln mit einer Base wieder abgespalten wird.

Die Kondensation der Verbindungen der Formeln IV und V findet wie diejenige der Verbindungen der Formeln II und III in Gegenwart der äquimolaren Menge einer Base als säurebindendes Mittel bei einer Temperatur von 0 °C bis zum Siedepunkt des Lösungsmittels statt.

Die Nitrierung des 3-(poly)Oxyalkylaminodiphenyläthers der Formel VI findet bei relativ milden Bedingungen statt, unter welchen der (poly)-Alkoxyaminorest erhalten bleibt z. B. mittels der 1,5 bis 2 molaren Menge 100 %iger Salpetersäure in Eisessig. Man kann den 3-(poly)-Oxyalkylaminodiphenyläther der Formel VI auch in Aethylenchlorid lösen, dazu vorsichtig mindestens die äquimolare Menge Schwefelsäure geben und dann unter Kühlen bei 0 bis 15° und Rühren langsam Nitriersäure (konz.

Schwefelsäure und konz. Salpetersäure 1 : 1) zutropfen lassen. Eine Modifikation besteht darin, dass man den 3-(poly)Oxyalkylaminodiphenyläther der Formel VI in Aethylenchlorid und mindestens der doppelt äquimolaren Menge Schwefelsäure auflöst, und dann unter Kühlen auf 0-15 °C und Rühren portionenweise Kaliumnitrat zugibt.

Man kann ein 3,4-Dichlorbenzotrifluorid der Formel V zuerst auch mit einem meta-Hydroxyanilin zum 3-Amino-2'-chlor-4'-trifluormethyldiphenyl-äther der Formel VII kondensieren,

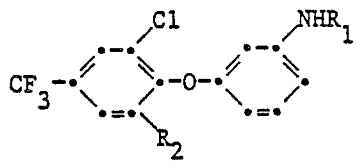

(VII)

worin $R_1$ Wasserstoff oder $C_1$-$C_4$-Alkyl bedeutet, und $R_2$ die unter Formel I gegebene Bedeutung hat, und dieses Produkt dann in einem inerten organischen Lösungsmittel, in Gegenwart einer Base, mit einem Polyoxyalkyloxyalkylhalid der Formel VIII umsetzt

$$X—(A—O)_n—(A'—O)_{n'}—(A''—O)_{n''}—(A'''—O)_{n'''}—R \qquad (VIII),$$

worin X ein Halogenatom, Methylsulfat oder den Tosylatrest bedeutet und A, A', A'', A''', n, n', n'', n''' und R die unter Formel I gegebene Bedeutung haben, und das entstandene Kondensationsprodukt, welches der Formel VI entspricht, mit Salpetersäure nitrieren, wobei falls $R_1$ Wasserstoff bedeutet, zuerst ein Acylrest $R_1'$ als Schutzgruppe eingeführt werden muss, der nach der Nitrierung durch Behandeln mit einer Base wieder abgespalten wird.

Als Schutzgruppen $R_1'$ kommen Acylreste, wie sie sich von niederen Fettsäuren, Alkylsulfonylsäuren, Benzolsulfonsäuren, Kohlensäurederivaten, oder Benzoesäuren ableiten, in Frage, die in Form ihrer Halide mit in den sekundär-Aminorest eingeführt werden und nach der Nitrierung durch kurzes Behandeln mit einer Base wie z. B. 1 oder 2 N Natriumhydroxyd oder Kaliumhydroxyd wieder abgespalten werden. Um die Schutzgruppe abzuspalten, genügt es im allgemeinen, in Gegenwart der Base kurz aufzukochen.

Schliesslich können die Verbindungen der Formel I, in denen R einen Alkylrest darstellt, auch hergestellt werden, indem man einen 2'-Chlor-4-nitro-3-(poly)hydroxyalkylamino-4'-trifluormethyldiphenyl-äther der Formel IX

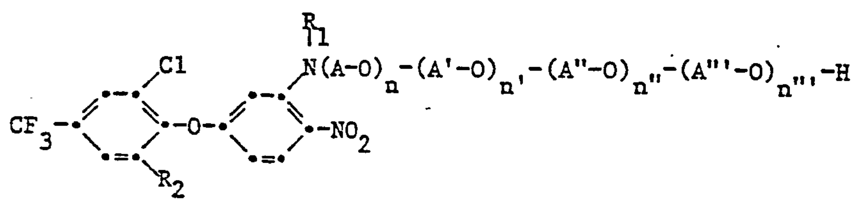

(IX)

worin A, A', A'' und A''', n, n', n'' und n''', $R_1$ und $R_2$ die unter Formel I gegebene Bedeutung haben, in einem inerten organischen Lösungsmittel in Gegenwart einer Base mit einem reaktionsfähigen Ester der Formel (X) umsetzt

$$X—R \qquad (X),$$

worin X ein Halogenatom, ein Sulfonsäure- oder Fettsäurerest bedeutet.

Bevorzugte Reste X sind Chlor, Brom, der Toluolsulfonsäurerest und z. B. ein niederer Alkylsulfonsäurerest.

Diese Kondensation oder Umsetzungen werden vorzugsweise in inerten organischen Lösungsmitteln wie z. B. Alkanolen, höhersiedenden Ketonen oder Aethern, in Aromaten, Amiden oder Sulfoxiden vorgenommen. Als Beispiele seien genannt Aethanol, Propanol, Isopropanol, Hexanol, Cyclohexanol, Methyläthylketon, Dioxan, Tetrahydrofuran, Benzol, Toluol, Dimethylformamid, Dimethylsulfoxid, Acetonitril.

Die Reaktionstemperaturen liegen vorzugsweise zwischen —20° und 120 °C. Die Umsetzungen verlaufen oft leicht exotherm und können bei Raumtemperatur durchgeführt werden. Zwecks Abkürzung der Reaktionszeit oder zum Einleiten der Reaktion wird zweckdienlich für kurze Zeit bis zum Siedepunkt des Reaktionsgemisches aufgewärmt.

Ausgangsstoffe der Formeln III und VIII werden hergestellt, ausgehend von den entsprechenden Monoäthern von poly-Alkylenglykolen, die man in Gegenwart von etwas Lösungsmittel und gegebenenfalls eines Katalysators mit einem Halogenierungsmittel, wie z. B. Thionylchlorid zum poly-Alkylenglykol-monoäther-Halogenid resp. Chlorid umsetzt, dieses gegebenenfalls aus der Reaktionsmasse durch

Destillieren isoliert oder gleich in einem Lösungsmittel, wie beispielsweise einem Alkanol oder Aether aufnimmt und im Autoklaven bei einer höheren Temperatur zwischen 50 und 200 °C und erhöhtem Druck mit Ammoniak umsetzt. Das Endprodukt wird durch Destillieren mit Benzol oder Toluol entwässert und dann durch eine fraktionierte Destillation gereinigt.

Die Verbindungen der Formel I haben gute herbizide Eigenschaften. In grossen Aufwandmengen können sie als Totalherbizide eingesetzt werden. Interessanter jedoch ist ihr Einsatz in Aufwandmengen von ca. 0,1 bis 5 kg pro Hektare als Selektivherbizide in Kulturpflanzungen, bei gekeimten im Nachauflaufverfahren oder bei frisch gesäten im Vorauflaufverfahren. Sie hemmen oder beeinträchtigen dort das Aufkommen von Unkräutern, zweikeimblättrigen, vor allem aber auch von vielen einkeimblättrigen Arten wie Lolium, Alopecurus, Rottboellia, Sorghum, Digitaria, Setaria und Panicum. Kulturpflanzen, wie Getreide, Gerste, Weizen, Roggen, Mais oder Reis aber auch andere wie Baumwolle und Soja werden dabei ganz oder mindestens bei der Aufwandmenge von 1 kg/ha geschont.

Die Verbindungen der Formel I können auch zur Bekämpfung Ektoparasiten, z. B. Lucilia sericata, sowie von Zecken an Haus- und Nutztieren, durch Tier-, Stall- und Weidebehandlung verwendet werden. Insbesondere eignen sie sich zur Bekämpfung von Insekten der Ordnungen Lepidoptera, Coleoptera, Homoptera, Heteroptera, Diptera, Thysanoptera, Orthoptera, Anoplura, Siphonaptera, Mallophage, Thysanura, Isoptera, Psocoptera und Hymenoptera sowie von Vertretern der Ordnung Akarina. Die pestizide Wirkung der erfindungsgemässen Verbindungen entspricht einer Abtötungsrate (Mortalität) von mindestens 50-60 % der erwähnten Schädlinge.

Ferner besitzen die Verbindungen der Formel I günstige wachstumsregulierende Effekte (Wuchshemmung). Es werden sowohl Monokkotyledonen wie auch Dikolyadonen in ihrem Wachstum beeinträchtigt. So können z. B. in der Landwirtschaft in tropischen Gegenden häufig als « cover crop » (Bodenbedecker) angepflanzte Leguminosen durch die Verbindungen der Formel I in ihrem Wachstum selektiv gehemmt werden, so dass zwar die Bodenerosion zwischen den Kulturpflanzen verhindert wird, die « cover crops » jedoch nicht zur Konkurrenz für die Kultur werden können.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : aromatische Kohlenwasserstoffe, bevorzugt die Fraktionen $C_8$-$C_{12}$, wie z. B. Xylolgemische oder substituierte Naphthaline, Phthalsäureester wie Dibutyl- oder Dikotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch

den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispielen nichtionische Tenside seien Nonylphenolpolyäthoxyäthanole, Ricinusölpolyglykoläther, Polypropylenpolyäthylenoxid-Addukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ridgewood, New Jersey, 1979 ;

J. and M. Ash, « Encyclopedia of Surfactants » Vol I-III, Chemical Publishing Co. Inc., New York, 1980/81

Dr. Helmut Stache, « Tensid Taschenbuch » Carl Hauser Verlag, München/Wien 1981.

Die herbiziden Zubereitungen enthalten in der Regel 0,1 bis 95 % insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen : ( % = Gewichtsprozent)

Emulgierbare Konzentrate

| | |
|---|---|
| Aktiver Wirkstoff : | 1 bis 20 %, bevorzugt 5 bis 10 % |
| oberflächenaktives Mittel : | 5 bis 30 %, vorzugsweise 10 bis 20 % |
| flüssiges Trägermittel : | 50 bis 94 %, vorzugsweise 70 bis 85 %. |

Stäube

| | |
|---|---|
| Aktiver Wirkstoff : | 0,1 bis 10 %, vorzugsweise 0,1 bis 1 % |
| festes Trägermittel : | 99,9 bis 90 %, vorzugsweise 99,9 bis 99 % |

Suspension-Konzentrate

| | |
|---|---|
| Aktiver Wirkstoff : | 5 bis 75 %, vorzugsweise 10 bis 50 % |
| Wasser : | 94 bis 25 %, vorzugsweise 90 bis 30 % |
| oberflächenaktives Mittel : | 1 bis 40 %, vorzugsweise 2 bis 30 %. |

Benetzbare Pulver

| | |
|---|---|
| Aktiver Wirkstoff : | 0,5 bis 90 %, vorzugsweise 1 bis 80 % |
| oberflächenaktives Mittel : | 0,5 bis 20 %, vorzugsweise 1 bis 15 % |
| festes Trägermaterial : | 5 bis 95 %, vorzugsweise 15 bis 90 %. |

Granulate

| | |
|---|---|
| Aktiver Wirkstoff : | 0,5 bis 30 %, vorzugsweise 3 bis 15 % |
| festes Trägermittel : | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C angegeben.

Beispiel 1

Herstellung von 3-(3,6-Dioxa-decylamino)-2'-chlornitro-4'-trifluormethyldiphenyl-äther

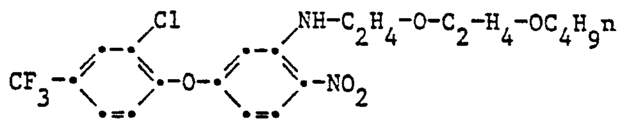

Zu einer Lösung von 9 g 2'-Chlor-3,4-dinitro-4'-trifluormethyldiphenyläther in 30 ml Dimethylsulfoxid gibt man unter Rühren 9 g 3,6-Dioxadecylamin und rührt bei Raumtemperatur während 10 Stunden. Dann wird das Reaktionsgemisch in Wasser gegossen und mit Aether extrahiert. Die Aetherphase wird erst mit verdünnter Salzsäure, dann mit Wasser gewaschen, über Natriumsulfat getrocknet, und eingedampft. Der Rückstand kristallisiert nach Zugabe von etwas Hexan. Man erhält so 7,3 g Titelprodukt mit Schmelzpunkt 73-74°.

In analoger Weise zu diesem Beispiel werden folgende Verbindungen erhalten :

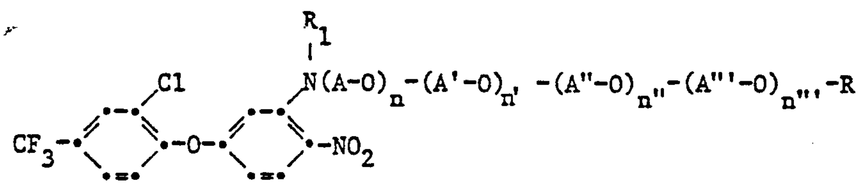

| No. | $R_1$ | $-(A-O)_n-(A'-O)_{n'}-(A''-O)_{n''}-(A'''-O)_{n'''}-R$ | phys. Konstante |
|---|---|---|---|
| 1 | H | $C_2H_4O-CH_3$ | Smp. 86-87° |
| 2 | H | $C_2H_4O-C_2H_5$ | Smp. 79-80° |
| 3 | H | $C_2H_4O-CH(CH_3)_2$ | Smp. 69-70° |
| 4 | H | $C_2H_4O-C_4H_9n$ | Smp. 73-75° |
| 5 | H | $C_2H_4O-CH_2CH(CH_3)_2$ | |
| 6 | H | $(C_2H_4O)_2-H$ | Smp. 70-72° |
| 7 | H | $(C_2H_4O)_2-CH_3$ | Smp. 49-50° |
| 8 | H | $(C_2H_4O)_2-C_2H_5$ | Smp. 82-84° |
| 9 | H | $(C_2H_4O)_2-C_3H_7n$ | |
| 10 | H | $(C_2H_4O)_2-C_4H_9n$ | Smp. 73-74° |
| 11 | H | $(C_2H_4O)_2-CH_2CH(CH_2)_2$ | |
| 12 | H | $(C_2H_4O)_3-H$ | Smp. 67-70° |
| 14 | H | $(C_2H_4O)_4-C_2H_5$ | Smp. 82-84° |
| 15 | $CH_3$ | $(C_2H_4O)_3-C_4H_9$ | |
| 16 | H | $C_2H_4O-C_2H_4O-C_2H_4O-CH(CH_3)_2$ | |

(Fortsetzung)

| No. | $R_1$ | $-(A-O)_n-(A'-O)_{n'}-(A''-O)_{n''}-(A'''-O)_{n'''}-R$ | phys. Konstante |
|---|---|---|---|
| 17 | H | $(C_2H_4O)_4-H$ | $n_D^{20}$ 1.5715 |
| 18 | H | $C_3H_6O-CH_3$ | Smp. 53-54° |
| 19 | H | $C_3H_6O-C_2H_5$ | Smp. 40-42° |
| 20 | H | $C_3H_6O-CH(CH_3)CH_2OH$ | |
| 21 | H | $C_3H_6O-CH(CH_3)CH_2O-C_2H_5$ | |
| 22 | H | $CH_2CH(CH_3)O-C_2H_4OH$ | |
| 23 | H | $CH_2CH(CH_3)O-C_2H_4O-CH_3$ | Oel $n_D^{21}$ 1.5756 |
| 24 | H | $CH_2CH(CH_3)O-C_2H_4O-C_2H_5$ | |
| 25 | H | $CH_2CH(CH_3)O-C_2H_4O-CH(CH_3)_2$ | |
| 26 | H | $CH_2CH(CH_3)O-C_2H_4O-C_4H_9n$ | |
| 27 | H | $CH_2CH(CH_3)O-C_3H_6OH$ | |
| 28 | H | $CH_2CH(CH_3)O-C_3H_6O-CH_3$ | |
| 29 | H | $CH_2CH(CH_3)O-C_3H_6O-C_4H_9n$ | |
| 30 | H | $CH_2CH(CH_3)O-(C_2H_4O)_2CH_3$ | Oel $n_D^{21}$ 1.5615 |
| 31 | H | $CH_2CH(CH_3)O-(C_2H_4O)_3CH_3$ | Oel $n_D^{21}$ 1.5522 |
| 32 | H | $CH_2CH(CH_3)O-(C_2H_4O)_2C_2H_5$ | Oel $n_D^{21}$ 1.5539 |
| 33 | H | $CH_2C(CH_3)_2OH$ | Smp. 78-80° |
| 34 | H | $[CH_2CH(CH_3)O]_2H$ | |
| 35 | H | $[CH_2CH(CH_3)O]_2C_2H_5$ | |
| 36 | H | $[CH_2CH(CH_3)O]_2C_4H_9n$ | |
| 37 | H | $[CH_2CH(CH_3)O]_2C_2H_4OCH_3$ | |
| 38 | H | $[CH_2CH(CH_3)O]_2C_2H_4OC_2H_5$ | |
| 39 | H | $[CH_2CH(CH_3)O]_2C_2H_4OC_4H_9n$ | |
| 40 | H | $[CH_2CH(CH_3)O]_2C_3H_6OH$ | |
| 41 | H | $[CH_2CH(CH_3)O]_2C_3H_6CH_3$ | |
| 42 | H | $[CH_2CH(CH_3)O]_3C_2H_4OH$ | |
| 43 | H | $[CH_2CH(CH_3)O]_3(C_2H_4O)_2CH_3$ | |
| 44 | H | $[CH_2CH(CH_3)O]_3(C_2H_4O)_2C_4H_9n$ | Oel $n_D^{21}$ 1.4983 |
| 45 | H | $[CH_2CH(CH_3)O]_3C_3H_6OC_2H_5$ | |
| 46 | H | $(C_2H_4O)_2CH_2CH(CH_3)OCH_3$ | Smp. 48-50° |
| 47 | H | $(C_2H_4O)_2CH_2CH(CH_3)OC_2H_5$ | |
| 48 | H | $(C_2H_4O)_2CH_2CH(CH_3)OC_4H_9$ | |
| 49 | H | $(C_2H_4O)_3CH_2CH(CH_3)OH$ | |
| 50 | H | $(C_2H_4O)_3CH_2CH(CH_3)OC_2H_5$ | |
| 51 | H | $(C_2H_4O)_3CH_2CH(CH_3)OCH_2CH(CH_3)_2$ | |
| 52 | H | $(C_2H_4O)_3[CH_2CH(CH_3)O]_2CH_3$ | |
| 53 | H | $(C_2H_4O)_4H$ | Smp. 111-113° |

(Fortsetzung)

| No. | $R_1$ | $-(A-O)_n-(A'-O)_{n'}-(A''-O)_{n''}-(A'''-O)_{n'''}-R$ | phys. Konstante |
|---|---|---|---|
| 54 | H | $(C_2H_4O)_4CH_3$ | |
| 55 | H | $(C_2H_4O)_4CH(CH_3)_2$ | |
| 56 | H | $(C_2H_4O)_4C_4H_9n$ | |
| 57 | H | $(C_2H_4O)_4CH_2CH(CH_3)OH$ | |
| 58 | H | $(C_2H_4O)_4CH_2CH(CH_3)OC_2H_5$ | |
| 59 | H | $(C_2H_4O)_4[CH_2CH(CH_3)O]_2H$ | |
| 60 | H | $(C_2H_4O)_4[CH_2CH(CH_3)O]_2CH_3$ | |
| 61 | $CH_3$ | $CH(CH_3)CH_2OCH_3$ | $n_D^{21}$ 1.5672 |
| 62 | $CH_3$ | $C_2H_4OCH_3$ | $n_D^{20}$ 1.5672 |
| 63 | $CH_3$ | $CH_2CH_2OCH(CH_3)CH_2OCH_3$ | |
| 64 | $CH_3$ | $C_3H_6OH$ | Smp. 113-115° |
| 65 | $CH_3$ | $C_3H_6OC_2H_5$ | |
| 66 | $CH_3$ | $CH_2CH(CH_3)OC_2H_4OCH_3$ | |
| 67 | $CH_3$ | $CH_2CH(CH_3)O-(C_2H_4O)_2CH_3$ | |
| 68 | $CH_3$ | $CH_2CH(CH_3)O-(C_2H_4O)_3CH_3$ | |
| 69 | $CH_3$ | $CH_2CH(CH_3)O-(C_2H_4O)_3C_2H_5$ | |
| 70 | $CH_3$ | $CH_2C(CH_3)_2OH$ | |
| 71 | $CH_3$ | $(CH_2CH_2O)_3H$ | |
| 72 | $CH_3$ | $(C_2H_4O)_3CH_3$ | $n_D^{21}$ 1.5509 |
| 73 | $CH_3$ | $(CH_2CH_2O)_3C_2H_5$ | |
| 74 | H | $CH(CH_3)CH_2O(C_2H_4O)_2CH_3$ | $n_D^{21}$ 1.5615 |
| 75 | H | $CH(CH_3)CH_2O(C_2H_4O)CH_3$ | $n_D^{21}$ 1.5756 |
| 76 | H | $CH(CH_3)CH_2O(C_2H_4O)_2C_2H_5$ | $n_D^{21}$ 1.5539 |
| 77 | H | $CH(CH_3)CH_2O(C_2H_4O)_3CH_3$ | $n_D^{21}$ 1.5522 |
| 78 | H | $CH(CH_3)CH_2O(C_2H_4O)_3C_4H_9n$ | $n_D^{23}$ 1.5450 |
| 79 | H | $CH_2CH(OCH_3)_2$ | Oel |
| 80 | H | $CH_2CH(OC_2H_5)_2$ | Smp. 73° |
| 81 | H | $C(CH_3)_2CH_2OH$ | Smp. 115-117° |
| 82 | H | $C(CH_3)_2CH_2OH$ | Smp. 76-79° |
| 83 | H | $C_5H_{10}OH$ | Oel |
| 84 | H | $CH(CH_3)CH_2OH$ | Smp. 78-79° |
| 85 | H | $[CH(CH_3)CH_2O]_2CH_3$ | $n_D^{21}$ 1.5702 |
| 86 | H | $(C_2H_4O)_2COCHCl_2$ | Oel |

(Fortsetzung)

| No. | $R_1$ | $-(A-O)_n-(A'-O)_{n'}-(A''-O)_{n''}-(A'''-O)_{n'''}-R$ | phys. Konstante |
|---|---|---|---|
| 87 | H | $(C_2H_4O)_2COCH_3$ | Smp. 63-65 |
| 88 | H | $(C_2H_4O)_2COCH_2COOCH_3$ | Oel |
| 89 | H | $(C_2H_4O)_2COCH_2Cl$ | Oel |
| 90 | H | $(C_2H_4O)_2COCCl_3$ | Oel |
| 91 | H | $CH_2CH(OH)CH_2OH$ | Smp. 118-120° |
| 92 | H | $CH_2CHOCOCH_3$<br>$CH_2OCOCH_3$ | Smp. 103-105° |
| 93 | H | $CH_2CH(OH)CH_2OCOCH_3$ | Harz |
| 94 | H | $(C_2H_4O)_3COCH_2COCH_3$ | Oel |
| 95 | H | $C_2H_4OCOCH_2CH_3$ | Smp. 67-71° |
| 96 | H | $CH_2CHOCOCH_2COCH_3$<br>$CH_2OCOCH_2COCH_3$ | Oel |
| 97 | H | $C_2H_4OC_2H_4COCH_3$ | Oel |
| 98 | H | $CH_2CH(CH_3)OCOCH_2COCH_3$ | Oel |
| 99 | H | $CH_2CH(CH_3)OCH_2OC_2H_4OCH_3$ | $n_D^{23}$ 1.5620 |
| 100 | H | $CH_2OC_2H_4OCH_3$ | Smp. 62-66° |
| 101 | H | $(C_2H_4O)_3CH_2OC_2H_4OCH_3$ | $n_D^{23}$ 1.5405 |
| 102 | H | $C_2H_4OCH_2OC_2H_4OCH_3$ | Smp. 50-52° |
| 103 | H | $(C_2H_4O)_2CH_2OC_2H_4OCH_3$ | Smp. 47-48° |
| 104 | H | $C(CH_3)_2CH_2OCOCH_2Cl$ | $n_D^{20}$ 1.5786 |
| 105 | H | $C_2H_4OC_2H_4CN$ | Smp. 77-79° |
| 106 | H | $(C_2H_4O)_3C_2H_4CN$ | Oel |
| 107 | H | $CH_2-CHO-CH$<br>$CH_2-O$ | Smp. 103 |
| 108 | H | $C_2H_4O-CH-O-CH(CH_3)$<br>$CH_2CH_2\ CH_2$ | Oel |
| 109 | H | $CH_2-CHCH_2O$<br>$OCH_2-CO$ | Smp. 120° Z |
| 110 | H | $CH_2-CHOCO$<br>$CH_2O$ | Harz |

(Fortsetzung)

| No. | $R_1$ | $-(A-O)_n-(A'-O)_{n'}-(A''-O)_{n''}-(A'''-O)_{n'''}$ | phys. Konst. |
|---|---|---|---|
| 111 | H | 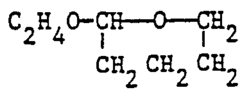 | Oel |
| 112 | 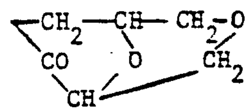 | | Smp. 133° |
| 113 | H | $(C_2H_4O)_2CO$ Phenyl | Oel |
| 114 | $COCH_2Cl$ | $CH_2OCH_3$ | Smp. 97° |
| 115 | $COCH_2Cl$ | $CH_2OC_2H_5$ | Smp. 112-114° |
| 116 | $C_2H_4OC_3H_7i$ | $C_2H_4OC_3H_7i$ | Oel |
| 117 | $C_2H_4OH$ | $(C_2H_4O)_3CH_3$ | $n_D^{21}$ 1.5356 |
| 118 | $COCH_2COCH_3$ | $CH_2CH(CH_3)OCOCH_2COCH_3$ | $n_D^{22}$ 1.5469 |
| 119 | H | $C_2H_4OSO_2$ para-Tolyl | Smp. 91-92° |
| 120 | H | 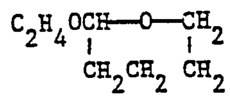 | Smp. 62° |

## Beispiel 2

Die als Ausgangsprodukte verwendeten Polyalkoxyamine werden wie folgt hergestellt:

a) 3,6,9-Trioxa-nonylamin $H_2N(C_2H_4O)_3H$

Man rührt im Autoklaven bei 130° 168,5 g 3,6-Dioxa-octanol-chlorhydrin in 150 ml Aethanol mit 150 g Ammoniak während 15 Stunden. Nach dem Erkalten versetzt man das Reaktionsgemisch langsam mit 43 g festes Natriumhydroxyd, filtriert und wäscht den festen Rückstand mit Alkohol. Das Filtrat wird eingedampft, durch azeotrope Destillation mit Toluol getrocknet und dann unter Vakuum fraktioniert. Man erhält 61 g 3,6,9-Trioxanonylamin, welches bei 102-106°/0,4 mbar siedet.

b) 3,6,9-Trioxa-undecylamin $H_2N—(CH_2—CH_2—O)_2C_2H_5$

Man rührt eine Lösung von 805 g 3,6-Dioxa-octanol in 10 ml Pyridin und tropft 487 ml Thionylchlorid zu. Nachdem alles zugetropft ist, wird die Mischung aufgeheizt, bis die Innentemperatur 80° erreicht hat. die anfangs lebhafte Gasentwicklung kommt nach ca. 3 Stunden zum Stillstand. Dann wird das Reaktionsgemisch unter Vakuum entgast. Es verbleiben 843 g 3,6-Dioxa-octyl-chlorid, welche in einem 1 Aethanol aufgenommen werden und zusammen mit 750 ml Ammoniak in einem Autoklav während 15 Stunden bei 130° gerührt werden. Nach dem Erkalten gibt man vorsichtig 240 g Natriumhydroxid dazu, filtriert das Ganze und wäscht den Filterrückstand mit Alkohol. Das Filtrat wird eingedampft und der Rückstand durch azeotrope Destillation mit Toluol getrocknet. Der Rückstand wird durch fraktionierte Destillation über eine Vigreux-Kolonne gereinigt. Die Hauptfraktion, das 3,6,9-Trioxa-undecylamin geht bei 73-76°/17 mbar über Ausbeute 382 g (48 % der Theorie).

Gemäss diesen Beispielen wurden folgende Amine hergestellt:

| | |
|---|---|
| $H_2N(C_2H_4O)_2CH_3$ | Sdp 70-74°/ mbar |
| $H_2N—CH_2CH(CH_3)O(C_2H_4O)_2CH_3$ | Sdp 78-79°/0.2 mbar |
| $H_2N(C_2H_4O)_3CH_3$ | Sdp 74-75°/0.5 mbar |
| $H_2N—CH_2CH(CH_3)O(C_2H_4O)CH_3$ | Sdp 98°/0.5 mbar |
| $H_2NCH_2CH(CH_3)OC_2H_4OCH_3$ | Sdp 70°/12 mbar |
| $H_2NCH_2CH(CH_3)O(C_2H_4O)_2C_2H_5$ | Sdp 70°/10,5 mbar |
| $H_2N(C_2H_4O)_2C_4H_9n$ | Sdp 95-97°/13 mbar |

Die durch Umsetzung des entsprechenden Alkylenglykols mit Thionylchlorid gemäss Beispiel b) erhaltenen Chloride können durch Destillation rein isoliert werden :

| | |
|---|---|
| $C1(C_2H_4O)_3CH_3$ | Sdp 75°/0.2 mbar |
| $C1CH_2CH(CH_3)OC_2H_4OCH_3$ | Sdp 75-80°/14 mbar |
| $C1CH_2CH(CH_3)O(C_2H_4O)_3CH_3$ | Sdp 125°/0.8 mbar |
| $C1CH_2CH(CH_3)O(C_2H_4O)_2C_2H_5$ | Sdp 125°/12 mbar |
| $C1(C_2H_4O)_2C_4H_9$ | Sdp 105°/18 mbar |

Die benötigten Alkylenglykole sind käuflich oder können nach dem folgenden Beispiel hergestellt werden.

c) 2-Methyl-3,6-dioxa-heptanol $CH_3O—C_2H_4OCH(CH_3)CH_2OH$

Eine Mischung von 310,6 g 3-Oxa-butanol, 232 g Propylenoxyd und 5 g festes Kaliumhydroxyd wird im Autoklaven während einer Stunde bei 70° gerührt. Nach dem Erkalten gibt man festes Kohlendioxyd zum Reaktionsgemisch, filtriert und destilliert über eine Fraktionierungskolonne. Die Hauptfraktion 2-Methyl-3,6-dioxa-heptanol siedet bei 79-85°/12 mbar Ausbeute 239 g.
Analog zu diesem Beispiel werden folgende Polyoxyäther hergestellt :

| | |
|---|---|
| $HOCH_2CH(CH_3)O(C_2H_4O)_2CH_3$ | Sdp 85°/0.3 mbar |
| $HOCH_2CH(CH_3)O(C_2H_4O)_3CH_3$ | Sdp 140°/0.8 mbar |
| $HOCH_2CH(CH_3)O(C_2H_4O)_2C_2H_5$ | Sdp 117°/12 mbar |

## Beispiel 3

Formulierungsbeispiele für Wirkstoffe der Formel I ( % = Gewichtsprozent)

| a) Spritzpulver | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsion-Konzentrat | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (78 Mol AeO) | 3 % |
| Wasser | 91 % |

Beispiel 4

Biologische Versuche zum Nachweis der Wirkung der Verbindungen der Formel I

Pre-emergente Herbizidwirkung (Vorauflaufwirkung)

Im Gewächshaus werden Pflanzensamen in Blumentöpfe von 12-15 cm Durchmesser gesät, so dass sich pro Topf 10-25 Pflänzchen entwickeln können. Unmittelbar nach der Einsaat wird die Erdoberfläche mit einer wässrigen Suspension der Wirkstoffe, erhalten aus einem 10 %igen Spritzpulver, behandelt. Es wird eine Konzentration angewendet, welche 4 kg Wirksubstanz pro Hektar entspricht. Die Blumentöpfe werden im Gewächshaus bei 22-25 °C und 50-70 % relativer Luftfeuchtigkeit und regelmässigem Begiessen gehalten und der Versuch nach 3 Wochen ausgewertet. Der Zustand der Pflanzen wurde gemäss dem folgenden Bewertungsschema registriert :

9 Pflanze gedeiht wie unbehandelte Kontrolle
6-9 leichte Schäden
5 mittlere Schäden
2-4 schwere Schäden
1 Pflanze abgestorben

Die Resultate sind wie folgt :

| Verbindung No. | 8 | 12 | 13 | 23 | 30 |
|---|---|---|---|---|---|
| Pflanze | | | | | |
| Avena sativa | 2 | 9 | 5 | 3 | 5 |
| Setaria italica | 1 | 2 | 1 | 1 | 1 |
| Sinapis alba | 1 | 1 | 1 | 1 | 1 |
| Stellaria media | 1 | 1 | 1 | 1 | 1 |

Post-emergente Herbizidwirkung (Nachauflaufwirkung)

Eine Anzahl Unkräuter und Kulturpflanzen, sowohl monocotyle wie dicotyle, wird nach dem Auflaufen (im 4- bis 6-Blattstadium) mit einer wässrigen Wirkstoffdispersersion in Dosierung von 4 kg Wirksubstanz pro Hektar auf die Pflanzen gespritzt und diese bei 24°-26° und 45-60 % rel. Luftfeuchtigkeit gehalten. 15 Tage nach Behandlung wird der Versuch nach derselben Notenskala wie im Preemergent-Versuch ausgeweitet.

Die Resultate sind wie folgt :

| Verbindung No. | 2 | | | | 18 | | | | 46 | | | | 76 | | | | 77 | | | |
|---|---|---|---|---|---|---|---|---|---|---|---|---|---|---|---|---|---|---|---|---|
| Aufwandmenge kg/h | 1 | $\frac{1}{2}$ | $\frac{1}{4}$ | $\frac{1}{8}$ | 1 | $\frac{1}{2}$ | $\frac{1}{4}$ | $\frac{1}{8}$ | 1 | $\frac{1}{2}$ | $\frac{1}{4}$ | $\frac{1}{8}$ | 1 | $\frac{1}{2}$ | $\frac{1}{4}$ | $\frac{1}{8}$ | 1 | $\frac{1}{2}$ | $\frac{1}{4}$ | $\frac{1}{8}$ |
| Pflanze | | | | | | | | | | | | | | | | | | | | |
| Gerste | 7 | 8 | 8 | 8 | 6 | 7 | 7 | 8 | 5 | 6 | 8 | 9 | 6 | 7 | 7 | 8 | 5 | 6 | 7 | 8 |
| Weizen | 7 | 8 | 8 | 8 | 5 | 6 | 7 | 8 | 5 | 8 | 9 | 9 | 7 | 8 | 8 | 8 | 5 | 6 | 7 | 8 |
| Mais | 6 | 7 | 8 | 8 | 7 | 8 | 8 | 9 | 4 | 7 | 8 | 8 | 7 | 7 | 8 | 8 | 6 | 7 | 7 | 8 |
| Hirse | 4 | 6 | 7 | 8 | 5 | 6 | 7 | 8 | 6 | 8 | 9 | 9 | 7 | 7 | 8 | 8 | 7 | 7 | 8 | 8 |
| Reis | 7 | 7 | 8 | 8 | 7 | 8 | 8 | 8 | 7 | 8 | 9 | 9 | 7 | 8 | 8 | 8 | 7 | 7 | 8 | 8 |
| Soja | 5 | 7 | 7 | 8 | 5 | 6 | 7 | 7 | 4 | 7 | 7 | 8 | 5 | 7 | 8 | 8 | 4 | 5 | 7 | 8 |
| Abutilon sp. | 1 | 1 | 2 | 3 | 1 | 1 | 1 | 3 | 1 | 2 | 2 | 2 | 1 | 1 | 3 | 3 | 1 | 1 | 1 | 3 |
| Amaranthus retrofl. | 1 | 1 | 1 | 1 | 1 | 1 | 1 | 1 | 1 | 1 | 1 | 1 | 1 | 1 | 2 | 3 | 1 | 1 | 1 | 1 |
| Chenopodium album | 1 | 1 | 1 | 1 | 1 | 1 | 1 | 1 | 1 | 1 | 1 | 1 | 1 | 1 | 2 | 2 | 1 | 1 | 1 | 2 |
| Solanum nigrum | 1 | 1 | 1 | 3 | 1 | 1 | 1 | 1 | 1 | 1 | 1 | 1 | 2 | 2 | 2 | 3 | 1 | 1 | 1 | 2 |
| Ipomoea | 1 | 1 | 1 | 1 | 1 | 1 | 1 | 1 | 1 | 1 | 2 | 2 | 1 | 1 | 1 | 1 | 1 | 1 | 1 | 1 |

(Fortsetzung)

| Verbindung No. | 2 | 18 | 46 | 76 | 77 |
|---|---|---|---|---|---|
| Aufwandmenge kg/h | 1 $\frac{1}{2}$ $\frac{1}{4}$ $\frac{1}{8}$ | 1 $\frac{1}{2}$ $\frac{1}{4}$ $\frac{1}{8}$ | 1 $\frac{1}{2}$ $\frac{1}{4}$ $\frac{1}{8}$ | 1 $\frac{1}{2}$ $\frac{1}{4}$ $\frac{1}{8}$ | 1 $\frac{1}{2}$ $\frac{1}{4}$ $\frac{1}{8}$ |
| Sinapis alba | 2 2 2 2 | 1 1 2 3 | 1 1 1 1 | 1 1 2 2 | 1 1 1 1 |
| Stellaria media | 2 2 4 4 | 1 1 2 3 | 1 1 3 4 | 1 2 2 4 | 1 1 3 4 |
| Chrysanthemum | 1 1 1 1 | 1 1 1 1 | 1 1 2 2 | 1 1 1 2 | 1 1 1 2 |
| Galium aparine | 2 3 4 4 | 1 2 3 3 | 2 2 3 3 | 1 1 2 3 | 2 3 4 4 |
| Viola tricolor | 1 1 1 1 | 1 1 1 1 | 1 1 1 1 | 1 1 1 1 | 1 1 1 2 |
| Veronika | 1 1 1 1 | 1 1 1 1 | 1 1 1 1 | 1 1 2 2 | 1 1 1 1 |
| Beta vulgaris | 3 3 4 4 | 1 3 3 4 | 2 2 3 3 | 2 3 4 4 | 1 1 3 4 |

Die Resultate sind wie folgt :

| Verbindung No. | 85 | 87 | 88 | 89 | 90 |
|---|---|---|---|---|---|
| Aufwandmenge kg/h | 1 $\frac{1}{2}$ $\frac{1}{4}$ $\frac{1}{8}$ | 1 $\frac{1}{2}$ $\frac{1}{4}$ $\frac{1}{8}$ | 1 $\frac{1}{2}$ $\frac{1}{4}$ $\frac{1}{8}$ | 1 $\frac{1}{2}$ $\frac{1}{4}$ $\frac{1}{8}$ | 1 $\frac{1}{2}$ $\frac{1}{4}$ $\frac{1}{8}$ |
| Pflanze | | | | | |
| Gerste | 5 7 7 8 | 7 8 9 9 | 8 8 9 9 | 2 8 8 8 | 8 8 8 9 |
| Weizen | 4 6 8 9 | 8 8 9 9 | 8 8 8 9 | 7 8 9 9 | 8 9 9 9 |
| Mais | 3 6 7 8 | 6 7 8 8 | 5 7 8 9 | 6 7 8 8 | 6 7 8 8 |
| Hirse | 5 7 8 9 | 4 6 7 7 | 6 7 7 9 | 6 7 7 8 | 5 6 7 7 |
| Reis | 7 8 9 9 | 7 8 8 9 | 8 9 9 9 | 7 7 9 9 | 8 9 9 9 |
| Soja | 4 6 7 8 | 4 5 6 7 | 6 7 7 8 | 6 7 7 9 | 3 6 6 6 |
| Abutilon sp. | 1 2 2 3 | 1 1 2 2 | 1 1 2 2 | 1 1 1 1 | 1 1 1 1 |
| Amaranthus retrofl. | 1 1 1 1 | 1 1 1 1 | 1 1 1 2 | 1 1 1 1 | 1 1 1 2 |
| Chenopodium album | 1 2 2 3 | 1 1 1 2 | 1 1 1 2 | 1 1 1 1 | 1 1 2 2 |
| Solanum nigrum | 1 1 1 2 | 1 1 1 1 | 1 1 3 3 | 1 1 1 1 | 1 1 1 1 |
| Ipomoea | 1 1 1 1 | 1 1 3 3 | 2 2 2 3 | 1 1 2 3 | 1 1 2 2 |
| Sinapis alba | 1 1 1 1 | 1 1 1 1 | 1 1 5 5 | 1 1 1 2 | 1 1 1 4 |
| Stellaria media | 1 3 3 5 | 2 3 3 3 | 2 2 3 4 | 2 2 3 4 | 1 2 3 4 |
| Chrysanthemum | 1 1 1 1 | 1 2 2 2 | 2 2 4 4 | 1 1 2 4 | 1 2 2 4 |
| Galium aparine | 2 2 2 2 | 2 2 2 2 | 2 2 3 4 | 2 2 2 6 | 2 2 2 4 |
| Viola tricolor | 1 1 1 1 | 1 1 1 1 | 1 1 1 1 | 1 1 1 1 | 1 1 1 1 |
| Veronika | 1 1 1 1 | 1 1 1 2 | 1 1 1 2 | 1 1 1 1 | 1 1 1 1 |
| Beta vulgaris | 2 3 3 3 | 3 3 3 4 | 3 3 4 5 | 3 3 3 3 | 3 3 4 4 |

Selektive herbizide Wirkung auf Reis im Nachauflaufverfahren

Reispflänzchen, welche 25 Tage alt sind, wurden im Gewächshaus in grosse rechteckige Eternitschalen verpflanzt. Zwischen die Reihen der Reispflanzen wurden dann Samen der in Reiskulturen

vorkommenden Unkräuter Echinochloa crus galli, Cyperus difformis, Ammania und Rotala gesät. Die Schalen wurden gut bewässert und bei einer Temperatur von ca. 25 °C und hoher Luftfeuchtigkeit gehalten. Nach 12 Tagen, wenn die Unkräuter aufgelaufen sind und das 2-3 Blattstadium erreicht haben, wurde die Erde in der Schale mit einer 2,5 cm hohen Schicht Wasser bedeckt. Der Wirkstoff wurde dann als Emulsionskonzentrat mittels einer Pipette zwischen die Pflanzenreihen appliziert, wobei man das Emulsionskonzentrat so verdünnt dass es einer Feld-Applikation von 1, 0.5, 0.25 oder 0.125 kg Aktivsubstanz pro Hektare entspricht. Der Versuch wird nach 4 Wochen ausgewertet, die Resultate sind untenstehend zusammengefasst.

| Verbindung No. | 18 | 46 | 76 | 77 | 85 |
|---|---|---|---|---|---|
| Aufwandmenge kg/ha | 1 $\frac{1}{2}$ $\frac{1}{4}$ $\frac{1}{8}$ | 1 $\frac{1}{2}$ $\frac{1}{4}$ $\frac{1}{8}$ | 1 $\frac{1}{2}$ $\frac{1}{4}$ $\frac{1}{8}$ | 1 $\frac{1}{2}$ $\frac{1}{4}$ $\frac{1}{8}$ | 1 $\frac{1}{2}$ $\frac{1}{4}$ $\frac{1}{8}$ |
| Pflanze | | | | | |
| Reis | 7 8 9 9 | 9 9 9 9 | 7 8 9 9 | 6 8 9 9 | 8 9 9 9 |
| Echinochloa crus g. | 1 1 1 1 | 1 1 2 4 | 1 1 1 2 | 1 1 1 3 | 1 3 4 5 |
| Scirpus s.p. | 1 1 1 1 | 1 1 1 2 | 2 2 3 3 | 1 1 1 1 | 1 1 1 4 |
| Monocharia vagin. | 1 1 1 1 | 1 1 1 1 | 1 1 1 1 | 1 1 1 1 | 1 1 1 2 |
| Sagittaria pygmea | 2 2 3 3 | 3 3 4 4 | 3 4 4 4 | 1 2 3 5 | 3 3 4 4 |

Wirkung gegen Lucilia sericata und cuprina

Zu 9 ml eines Zuchtmediums wird bei 50 °C ein ml einer 0,5 % Aktivsubstanz enthaltenden wässrigen Zubereitung hinzugefügt. Nun werden ca. 30 frisch geschlüpfte Lucilia Larven zum Zuchtmedium gegeben. Nach 48 und 96 Stunden wird die insektizide Wirkung durch Ermittlung der Abtöthungsrate festgestellt.

Verbindungen der Formel I zeigen in diesem Test gute Wirkung gegen die geprüften Lucilia Arten.

Wirkung gegen Aëdes aegypti

Auf die Oberfläche von 150 ml Wasser, das sich in einem Behälter befindet, wird so viel einer 0,1 %igen acetonischen Lösung des Wirkstoffs pipettiert, dass eine Konzentration von 12,5 ppm erhalten wird. Nach Verdunsten des Acetons wird der Behälter mit 30 bis 40 2-tägigen Aëdes-Larven beschickt. Nach 2 und 7 Tagen wird die % Mortalität (Anzahl der schwimmun fähigen Larven) geprüft.

Verbindungen der Formel I zeigen in diesem Test gute Wirkung gegen Aëdes aegypti.

**Patentansprüche**

1. 3-(poly)Oxyalkylamino-2'-chlor-4-nitro-4'-trifluormethyldiphenyläther der Formel

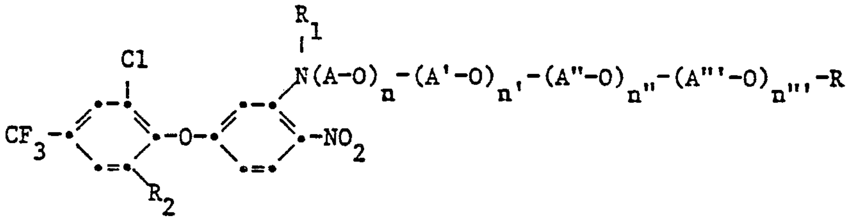

(I)

worin A, A', A" A"' je ein gleicher oder verschiedender $C_1$-$C_4$-Alkylenrest der geradkettig oder verzweigt sein kann,

n eine Zahl 1 bis 5

n', n" n"' je Null oder eine Zahl von 1 bis 5 und

R ein $C_1$-$C_4$-Alkylrest, ein $C_1$-$C_4$-Alkylcarbonylrest, wobei die Alkylreste durch Halogen oder Cyan substituiert sein können ; ein Benzoyl- oder Phenylsulfonylrest der durch Halogen, $C_1$-$C_4$-Alkyl, $C_1$-$C_4$-Alkoxy, $C_1$-$C_4$-Halogenalkyl, $C_1$-$C_4$-Halogenalkoxy oder Nitro substituiert sein kann, oder falls A—O nicht

Aethylenoxy und/oder falls die Summe von n, n', n" und n"' grösser als 1 ist, auch Wasserstoff bedeuten,

$R_1$ Wasserstoff, $C_1$-$C_4$-Alkyl, $C_1$-$C_4$-Hydroxyalkyl, $C_2$-$C_8$-Alkoxyalkyl, $C_1$-$C_4$-Alkylcarbonyl oder $C_1$-$C_4$-Halogenalkylcarbonyl, wobei die Reste R und $R_1$ über C—C oder C—O-Bindungen verzweigt oder gegebenenfalls auch miteinander ringgeschlossen sein können, und

$R_2$ Wasserstoff, Chlor oder Fluor bedeuten.

2. 3-(poly-)Oxyalkylamino-2'-chlor-4-nitro-4'-trifluormethyldiphenyläther der Formel I, Anspruch 1, worin die Summe von n, n', n" und n"' grösser als 1 ist während A, A', A", A"', R, $R_1$ und $R_2$ die im Anspruch 1 gegebene Bedeutung haben.

3. 3-(poly-)Oxyalkylamino-2'-chlor-4-nitro-4'-trifluormethyldiphenyläther der Formel I gemäss Anspruch 1, worin A, A', A" und A"' je ein Aethylen, 1-Methyläthylen oder Propylenrest und $R_1$ Wasserstoff bedeuten, während n, n', n", n"', R und $R_2$ die im Anspruch 1 gegebene Bedeutung haben.

4. 3-(poly-)Oxyalkylamino-2'-chlor-4-nitro-4'-trifluormethyldiphenyläther der Formel I gemäss Anspruch 1, worin A, A', A" und A"' je einen Aethylen- oder 1-Methyläthylenrest bedeuten, während n, n', n", n"', R, $R_1$ und $R_2$ die in Anspruch 1 gegebene Bedeutung haben.

5. 3-(poly-)Oxyalkylamino-2'-chlor-4-nitro-4'-trifluormethyldiphenyläther der Formel I gemäss Anspruch 1, worin A, A', A" und A"' je einen Aethylenrest bedeuten, während n, n', n", n"', R, $R_1$ und $R_2$ die im Anspruch 1 gegebene Bedeutung haben.

6. 3-(3,6-Dioxa-octylamino)-2'-chlor-4-nitro-4'-trifluormethyldiphenyläther gemäss Anspruch 1.

7. 3-(3-Oxaheptylamino)-2'-chlor-4-nitro-4'-trifluormethyldiphenyläther gemäss Anspruch 1.

8. 2'-Chlor-3-(3,6,9-trioxa-decylamino)-4-nitro-4'-trifluormethyldiphenyläther gemäss Anspruch 1.

9. 2'-Chlor-4-nitro-3-(3,6,9-trioxanonylamino)-4'-trifluormethyldiphenyläther gemäss Anspruch 1.

10. 2'-Chlor-3-(4,7,10-trioxa-undec-2-ylamino)-4-nitro-4'-trifluormethyldiphenyläther gemäss Anspruch 1.

11. 2'-Chlor-3-(4,7-dioxa-oct-2-ylamino)-4-nitro-4'-trifluormethyldiphenyläther gemäss Anspruch 1.

12. 3-(3,6-Dioxa-decylamino)-2'-chlor-4-nitro-4'-trifluormethyldiphenyläther gemäss Anspruch 1.

13. 3-(4,7,10-Trioxa-dodec-2-ylamino)-2'-chlor-4-nitro-4'-trifluormethyldiphenyläther gemäss Anspruch 1.

14. 2'-Chlor-3-(4,7,10,13-tetraoxa-tetradec-2-ylamino)-4-nitro-4'-trifluormethyldiphenyläther gemäss Anspruch 1.

15. 3-(2,5,8-Trimethyl-3,6,9,12,15-pentaoxa-nonadecylamino)-2'-chlor-4-nitro-4'-trifluormethyldiphenyläther gemäss Anspruch 1.

16. Verfahren zur Herstellung der 3-(poly-)Oxyalkylamino-2'-chlor-4-nitro-4'-trifluormethyldiphenyläther der Formel I, Anspruch 1, dadurch gekennzeichnet, dass man einen 2'-Chlor-3,4-dinitro-4'-trifluormethyldiphenyläther der Formel II

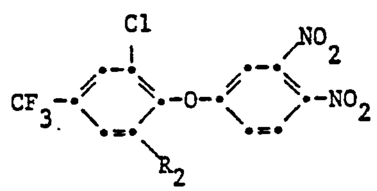

(II)

worin $R_2$ Wasserstoff, Chlor oder Fluor bedeutet, in einem inerten organischen Lösungsmittel in Gegenwart der molaren Menge eines säurebindenden Mittels, mit einem (poly-)Alkoxyamin der Formel III

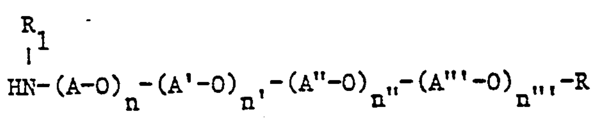

(III)

umsetzt, worin A, A', A", A"', n, n', n", n"', R und $R_1$ die im Anspruch 1 gegebene Bedeutung haben.

17. Verfahren zur Herstellung der 3-(poly-)Oxyalkylamino-2'-chlor-4-nitro-4'-trifluormethyldiphenyläther der Formel I, Anspruch 1, dadurch gekennzeichnet, dass man einen 3-(poly-)Oxyalkylaminophenol der Formel IV

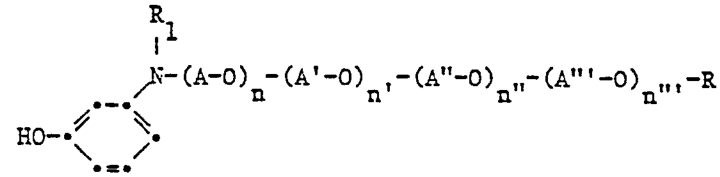

(IV)

worin A, A', A", A"', n, n', n", n"', R und $R_1$ die im Anspruch 1 gegebene Bedeutung haben, zuerst in einem inerten organischen Lösungsmittel in Gegenwart der molaren Menge eines säurebindenden Mittels mit einem 1,2-Dichlor-4-trifluormethylbenzol der Formel V

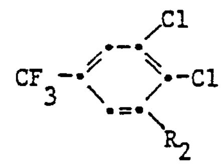

(V)

worin $R_2$ Wasserstoff, Chlor oder Fluor bedeutet, kondensiert, und das Kondensationsprodukt der Formel VI

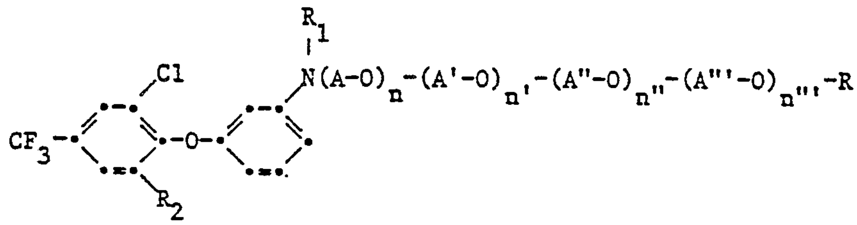

(VI)

worin A, A', A'', A''', n, n', n'', n''', R, $R_1$ und $R_2$ die im Anspruch 1 gegebene Bedeutung haben, anschliessend mit Salpetersäure nitriert, wobei falls $R_1$ Wasserstoff bedeutet, zuerst ein Acylrest $R_1'$ als Schutzgruppe eingeführt werden muss, der nach der Nitrierung durch Behandeln mit einer Base wieder abgespalten wird.

18. Verfahren zur Herstellung der 3-(poly-)Oxyalkylamino-2'-chlor-4-nitro-4'-trifluormethyldiphenyläther der Formel I, Anspruch 1, dadurch gekennzeichnet, dass man 3-Amino-2'-chlor-4'-trifluormethyldiphenyläther der Formel VII

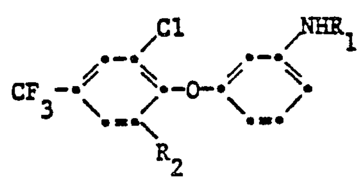

(VII)

worin $R_1$ Wasserstoff oder $C_1$-$C_4$-Alkyl und $R_2$ Wasserstoff, Chlor oder Fluor bedeuten, in einem inerten organischen Lösungsmittel in Gegenwart der molaren Menge eines säurebindenden Mittels mit einem Polyalkylenoxyalkylhalid der Formel VIII

$$X-(A-O)_n-(A'-O)_{n'}-(A''-O)_{n''}-(A'''-O)_{n'''}-R \quad (VIII)$$

kondensiert, worin X ein Halogenatom, Methylsulfat oder Tosylat bedeutet und A, A', A'', A''', n, n', n'', n''' und R die im Anspruch 1 gegebene Bedeutung haben und das Kondensationsprodukt der Formel VI

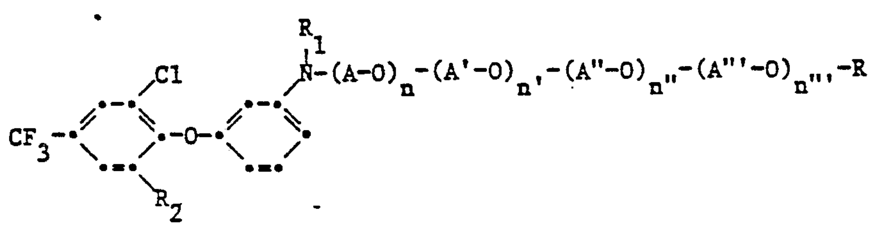

(VI)

worin A, A', A'', A''', n, n', n'', n''', R, $R_1$ und $R_2$ die im Anspruch 1 gegebene Bedeutung haben, anschliessend mit Salpetersäure nitriert, wobei falls $R_1$ Wasserstoff bedeutet, zuerst ein Acylrest $R_1'$ als Schutzgruppe eingeführt werden muss, der nach der Nitrierung durch Behandeln mit einer Base wieder abgespalten wird.

19. Herbizides Mittel, dadurch gekennzeichnet, dass es neben inerten Zutaten als Wirkstoff einen 3-(poly-)Oxyalkylamino-2'-chlor-4-nitro-4'-trifluormethyldiphenyläther der Formel I gemäss Anspruch 1 enthält.

20. Die Verwendung der 3-(poly-)Oxyalkylamino-2'-chlor-4-nitro-4'-trifluormethyldiphenyläther gemäss Anspruch 1 zur selektiven Unkrautbekämpfung in Nutzpflanzenkulturen.

21. Die Verwendung der 3-(poly-)Oxyalkylamino-2'-chlor-4-nitro-4'-trifluormethyldiphenyläther gemäss Anspruch 1 zur selektiven Unkrautbekämpfung in Kulturen von Getreide, Mais, Reis und Soja.

22. Die Verwendung der 3-(poly-)Oxyalkylamino-2'-chlor-4-nitro-4'-trifluormethyldiphenyläther gemäss Anspruch 1 zur Regulierung des Pflanzenwachstums.

23. Verfahren zur Bekämpfung von Unkräutern in Nutzpflanzenkulturen, dadurch gekennzeichnet,

dass man die Kulturen oder deren Anbaugebiet mit einer wirksamen Menge eines 3-(poly-)Oxyalkylamino-2'-chlor-4-nitro-4'-trifluormethyldiphenyläthers gemäss Anspruch 1, behandelt.

24. Verfahren zur Regulierung des Pflanzenwuchses, dadurch gekennzeichnet, dass man die Pflanzen oder Pflanzenteile, deren Wachstum man regulieren möchte, mit einer wirksamen Menge eines 3-(poly-)Oxyalkylamino-2'-chlor-4-nitro-4'-trifluormethyldiphenyläther gemäss Anspruch 1 behandelt.

25. Die Verwendung der 3-(poly)-Oxyalkylamino-2'-chlor-4-nitro-4'-trifluormethyldiphenyläther gemäss Anspruch 1 zur Bekämpfung von Insekten und Vertretern der Ordnung Akarina.

## Claims

1. A 3-(poly)oxyalkylamino-2'-chloro-4-nitro-4'-trifluoromethyldiphenyl ether of the formula

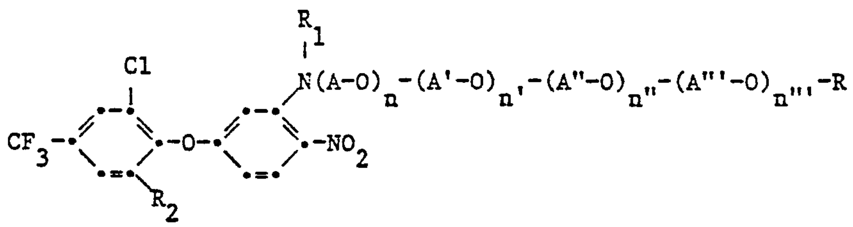

(I)

wherein each of A, A', A″ and A‴ is an identical or different $C_1$-$C_4$alkylene radical which may be straight chain or branched ;

n is a value from 1 to 5 ;

each of n', n″ and n‴ is zero or a value from 1 to 5 ;

R is a $C_1$-$C_4$alkyl radical or a $C_1$-$C_4$alkylcarbonyl radical, which alkyl radicals may be substituted by halogen or cyano, or is a benzoylsulfonyl or phenylsulfonyl radical which may be substituted by halogen, $C_1$-$C_4$alkyl, $C_1$-$C_4$alkoxy, $C_1$-$C_4$haloalkyl, $C_1$-$C_4$haloalkoxy or nitro, or, if A—O is not oxyethylene and/or if the sum of n + n' + n″ + n‴ is not greater than 1, R is also hydrogen ;

$R_1$ is hydrogen, $C_1$-$C_4$alkyl, $C_1$-$C_4$hydroxyalkyl, $C_2$-$C_8$alkoxyalkyl, $C_1$-$C_4$alkylcarbonyl or $C_1$-$C_4$haloalkylcarbonyl ; which radicals R and

$R_1$ may be branched through C—C or C—O bonds or may be linked to each other to form a ring ;

and $R_2$ is hydrogen, chlorine or fluorine.

2. A 3-(poly)oxyalkylamino-2'-chloro-4-nitro-4'-trifluoromethyldiphenyl ether of formula I according to claim 1, wherein the sum of n + n' + n″ + n‴ is greater than 1, and A, A', A″, A‴, R, $R_1$ and $R_2$ are as defined in claim 1.

3. A 3-(poly)oxyalkylamino-2'-chloro-4-nitro-4'-trifluoromethyldiphenyl ether of formula I according to claim 1, wherein each of A, A', A″ and A‴ is an ethylene, 1-methylethylene or propylene radical, $R_1$ is hydrogen, and n, n', n″, n‴, R and $R_2$ are as defined in claim 1.

4. A 3-(poly)oxyalkylamino-2'-chloro-4-nitro-4'-trifluoromethyldiphenyl ether of formula I according to claim 1, wherein each of A, A', A″ and A‴ is an ethylene or 1-methylethylene radical, and n, n', n″, n‴, R, $R_1$ and $R_2$ are as defined in claim 1.

5. A 3-(poly)oxyalkylamino-2'-chloro-4-nitro-4'-trifluoromethyldiphenyl ether of formula I according to claim 1, wherein each of A, A', A″ and A‴ is an ethylene radical, and n, n', n″, n‴, R, $R_1$ and $R_2$ are as defined in claim 1.

6. 3-(3,6-dioxa-octylamino)-2'-chloro-4-nitro-4'-trifluoromethyldiphenyl ether according to claim 1.

7. 3-n-(3-oxaheptylamino)-2'-chloro-4-nitro-4'-trifluoromethyldiphenyl ether according to claim 1.

8. 2'-Chloro-3-(3,6,9-trioxa-decylamino)-4-nitro-4'-trifluoromethyldiphenyl ether according to claim 1.

9. 2'-Chloro-4-nitro-3-(3,6,9-trioxa-nonylamino)-4'-trifluoromethyldiphenyl ether according to claim 1.

10. 2'-Chloro-3-(4,7,10-trioxa-undec-2-ylamino)-4-nitro-4'-trifluoromethyldiphenyl ether according to claim 1.

11. 2'-Chloro-3-(4,7-dioxa-oct-2-ylamino)-4-nitro-4'-trifluoromethyldiphenyl ether according to claim 1.

12. 3-(3,6-dioxa-decylamino)-2'-chloro-4-nitro-4'-trifluoromethyldiphenyl ether according to claim 1.

13. 3-(4,7,10-trioxa-dodec-2-ylamino)-2'-chloro-4-nitro-4'-trifluoromethyldiphenyl ether according to claim 1.

14. 2'-Chloro-3'-(4,7,10,13-tetraoxa-tetradec-2-ylamino)-4-nitro-4'-trifluoromethyldiphenyl ether according to claim 1.

15. 3-(2,5,8-trimethyl-3,6,9,12,15-pentaoxanonadecylamino)-2'-chloro-4-nitro-4-trifluoromethyldiphenyl ether according to claim 1.

16. A process for the preparation of a 3-(poly)oxyalkylamino-2'-chloro-4-nitro-4'-trifluoromethyldiphenyl ether of formula I according to claim 1, which process comprises reacting a 2'-chloro-3,4-dinitro-4'-trifluoromethyldiphenyl ether of formula II

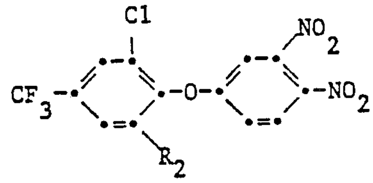

(II)

wherein $R_2$ is hydrogen, chlorine or fluorine, in an inert organic solvent and in the presence of the molar amount of an acid acceptor, with a (poly)alkoxyamine of formula III

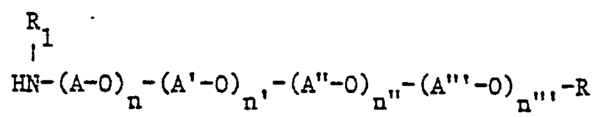

(III)

wherein A, A', A'', A''', n, n', n'', n''', R and $R_1$ are as defined in claim 1.

17. A process for the preparation of a 3-(poly)oxyalkylamino-2'-chloro-4-nitro-4'-trifluoromethyldiphenyl ether of formula I according to claim 1, which comprises first condensing a 3-(poly)oxyalkylaminophenol of formula IV

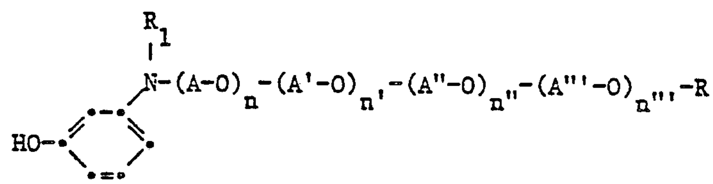

(IV)

wherein A, A', A'', A''', n, n', n'', n''', R and $R_1$ are as defined in claim 1, in an inert organic solvent and in the presence of the molar amount of an acid acceptor, with a 1,2-dichloro-4-trifluoromethylbenzene of formula V

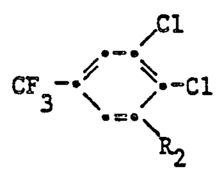

(V)

wherein $R_2$ is hydrogen, chlorine or fluorine, and subsequently nitrating the condensate of formula VI

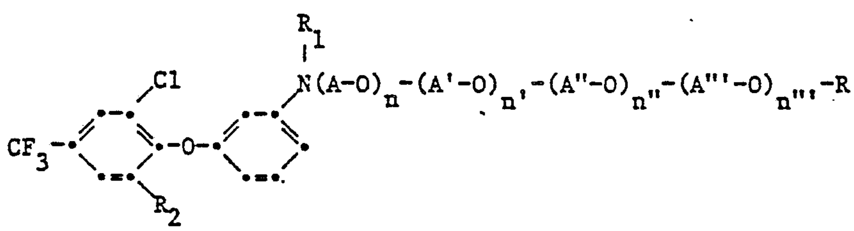

(VI)

wherein A, A', A'', A''', n, n', n'', n''', R, $R_1$ und $R_2$ are as defined in claim 1, with nitric acid, with the proviso that if $R_1$ is hydrogen, an acyl radical $R_1'$ must first be introduced as protecting group, which acyl radical is removed after the nitration by treatment with a base.

18. A process for the preparation of a 3-(poly)oxyalkylamino-2'-chloro-4-nitro-4'-trifluoromethyldiphenyl ether of formula I according to claim 1, which process comprises condensing a 3-amino-2'-chloro-4'-trifluoromethyldiphenyl ether of formula VII

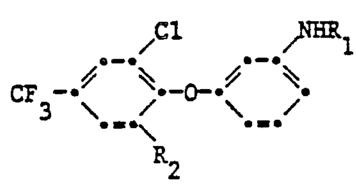

(VII)

wherein $R_1$ is hydrogen or $C_1$-$C_4$alkyl and $R_2$ is hydrogen, chlorine or fluorine, in an inert organic solvent and in the presence of the molar equivalent of an acid acceptor, with a polyoxyalkyloxyalkyl halide of formula VIII

$$X(A—O)_n—(A'—O)_{n'}—(A''—O)_{n''}—(A'''—O)_{n'''}—R \quad (VIII),$$

wherein X is a halogen atom, methyl sulfate or the tosylate radical and A, A', A'', A''', n, n', n'', n''' and R are as defined in claim 1, and subsequently nitrating the resultant condensate of formula VI

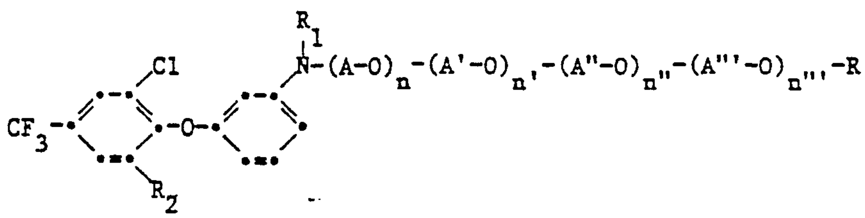

(VI)

wherein A, A', A'', A''', n, n', n'', n''', R, $R_1$ und $R_2$ are as defined in claim 1, with nitric acid, with the proviso that if $R_1$ is hydrogen, an acyl radical $R_1'$ must first be introduced as protecting group, which acyl radical is removed after the nitration by treatment with a base.

19. A herbicidal composition which contains a 3-(poly)oxyalkylamino-2'-chloro-4-nitro-4'-trifluoromethyldiphenyl ether of formula I according to claim 1, together with inert adjuvants.

20. Use of a 3-(poly)oxyalkylamino-2'-chloro-4-nitro-4'-trifluoromethyldiphenyl ether according to claim 1 for selectively controlling weeds in crops of useful plants.

21. Use of a 3-(poly)oxyalkylamino-2'-chloro-4-nitro-4'-trifluoromethyldiphenyl ether according to claim 1 for selectively controlling weeds in crops of maize, rice and soybeans.

22. Use of a 3-(poly)oxyalkylamino-2'-chloro-4-nitro-4'-trifluoromethyldiphenyl ether according to claim 1 for regulating plant growth.

23. A method of selectively controlling weeds in crops of useful plants, which method comprises treating the crops or the crop area with a herbicidally effective amount of a 3-alkoxyamino- or a 3-polyalkoxyamino-2'-chloro-4-nitro-4'-trifluoromethyldiphenyl ether according to claim 1.

24. A method of regulating plant growth, which method comprises treating the plants or the parts of plants whose growth it is desired to regulate with an effective amount of a 3-alkoxyamino- or 3-polyalkoxyamino-2'-chloro-4-nitro-4'-trifluoromethyldiphenyl ether according to claim 1.

25. A method of controlling insects and representatives of the order Acarina, which method comprises the use of an effective amount of a 3-alkoxyamino- or a 3-polyalkoxyamino-2'-chloro-4-nitro-4'-trifluoromethyldiphenyl ether according to claim 1.

**Revendications**

1. 3-(poly)oxyalcoylamino-2'-chloro-4-nitro-4'-trifluorométhyldiphényléthers de formule

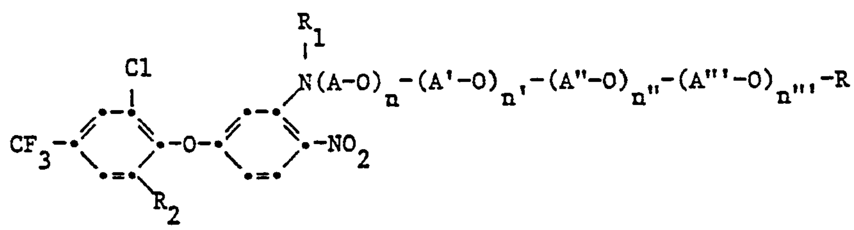

(I)

où A, A', A'' et A''' représentent chacun un radical alcoylène en $C_1$ à $C_4$ identique ou différent qui peut être à chaîne droite ou ramifiée,

n représente un nombre allant de 1 à 5,

n', n'', n''' valent chacun 0 ou un nombre allant de 1 à 5 et

R représente un radical alcoyle en $C_1$ à $C_4$, un radical carbonyle en $C_1$ à $C_4$, où les radicaux alcoyle peuvent être substitués par halogène ou cyano ; un radical benzoyle ou phénylsulfonyle qui peut être substitué par halogène, alcoyle en $C_1$ à $C_4$, alcoxy en $C_1$ à $C_4$, halogène-alcoyle en $C_1$ à $C_4$, halogène-alcoxy en $C_1$ à $C_4$ ou nitro, ou si A—O ne représente pas un éthylènoxy et/ou si la somme de n, n', n'' et n''' est supérieure à 1, également un hydrogène,

$R_1$ représente un hydrogène, alcoyle en $C_1$ à $C_4$, hydroxy-alcoyle en $C_1$ à $C_4$, alcoxy-alcoyle en $C_2$ à $C_8$, alcoyl-carbonyle en $C_1$ à $C_4$ ou halogène-alcoylcarbonyle en $C_1$ à $C_4$ où les radicaux R et $R_1$ peuvent être ramifiés par l'intermédiaire de liaison C—C ou C—O ou le cas échéant éventuellement cyclisés l'un avec l'autre et

$R_2$ représente un hydrogène, chlore ou fluor.

2. 3-(poly-)oxyalcoylamino-2'-chloro-4-nitro-4'-trifluorométhyldiphényléthers de formule I, de la

revendication 1, où la somme de n, n', n'' et n''' est supérieure à 1 tandis que A, A', A'', A''', R, $R_1$ et $R_2$ ont la signification donnée dans la revendication 1.

3. 3-(poly-)oxyalcoylamino-2'-chloro-4-nitro-4'-trifluorométhyldiphényléthers de formule I selon la revendication 1, où A, A', A'' et A''' représentent chacun un radical éthylène, 1-méthyléthylène ou propylène et $R_1$ représente un hydrogène, tandis que n, n', n'', n''', R et $R_2$ ont la signification donnée dans la revendication 1.

4. 3-(poly-)oxyalcoylamino-2'-chloro-4-nitro-4'-trifluorométhyldiphényléthers de formule I selon la revendication 1, où A, A', A'' et A''' représentent chacun un radical éthylène, ou 1-méthyléthylène, tandis que n, n', n'', n''', R, $R_1$ et $R_2$ ont la signification donnée dans la revendication 1.

5. 3-(poly-)oxyalcoylamino-2'-chloro-4-nitro-4'-trifluorométhyldiphényléthers de formule I selon la revendication 1, où A, A', A'' et A''' représentent chacun un radical éthylène, tandis que n, n', n'' et n''', $R_1$ et $R_2$ ont la signification donnée dans la revendication 1.

6. 3-(3,6-dioxa-octylamino)-2'-chloro-4-nitro-4'-trifluorométhyldiphényléther selon la revendication 1.

7. 3-(3-oxaheptylamino)-2'-chloro-4-nitro-4'-trifluorométhyldiphényléther selon la revendication 1.

8. 2'-chloro-3-(3,6,9-trioxa-décylamino)-4-nitro-4'-trifluorométhyldiphényléther selon la revendication 1.

9. 2'-chloro-4-nitro-3-(3,6,9-trioxanonylamino)-4'-trifluorométhyldiphényléther selon la revendication 1.

10. 2'-chloro-3-(4,7,10-trioxa-undéc-2-ylamino)-4-nitro-4'-trifluorométhyldiphényléther selon la revendication 1.

11. 2'-chloro-3-(4,7-dioxa-oct-2-ylamino)-4-nitro-4'-trifluorométhyldiphényléther selon la revendication 1.

12. 3-(3,6-dioxa-décylamino)-2'-chloro-4-nitro-4'-trifluorométhyldiphényléther selon la revendication 1.

13. 3-(4,7,10-trioxa-dodéc-2-ylamino)-2'-chloro-4-nitro-4'-trifluorométhyldiphényléther selon la revendication 1.

14. 2'-chloro-3-(4,7,10,13-tétraoxa-tétradéc-2-ylamino)-4-nitro-4'-trifluorométhyldiphényléther selon la revendication 1.

15. 3-(2,5,8-triméthyl-3,6,9,12,15-pentaoxa-nonadécylamino)-2'-chloro-4-nitro-4'-trifluorométhyldiphényléther selon la revendication 1.

16. Procédé de préparation du 3-(poly-)oxyalcoylamino-2'-chloro-4-nitro-4'-trifluorométhyldiphényléther de formule I, de la revendication 1, caractérisé en ce qu'on fait réagir un 2'-chloro-3,4-dinitro-4'-trifluorométhyldiphényléther de formule II

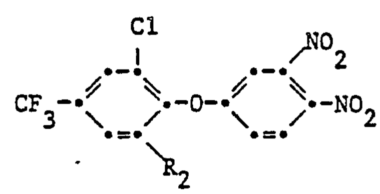

(II)

où $R_1$ représente un hydrogène, chlore ou fluor, dans un solvant organique inerte en présence d'une quantité molaire d'un agent liant d'acide, avec une (poly-)alcoxyamine de formule III

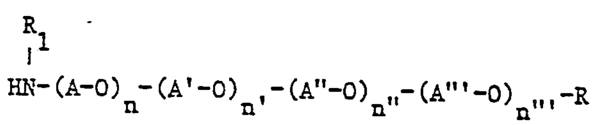

(III)

où A, A', A'', A''', n, n', n'', n''', R et $R_1$ ont la signification donnée dans la revendication 1.

17. Procédé de préparation du 3-(poly-)oxyalcoylamino-2'-chloro-4-nitro-4'-trifluorométhyldiphényléther de formule I, de la revendication 1, caractérisé en ce qu'on condense tout d'abord un 3-(poly-)oxyalcoylaminophénol de formule IV

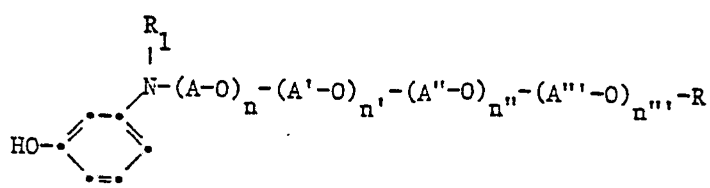

(IV)

où A, A', A'', A''', n, n', n'', n''', R et $R_1$ ont la signification donnée dans la revendication 1, dans un solvant organique inerte en présence d'une quantité molaire d'un agent liant d'acide avec un 1,2-dichloro-4-trifluorométhylbenzène de formule V

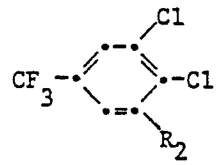

(V)

où $R_2$ représente un hydrogène, chlore ou fluor, et en ce qu'on nitre ensuite avec de l'acide nitrique le produit de condensation de formule VI

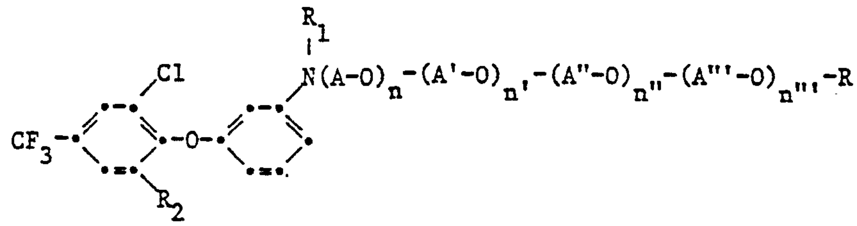

(VI)

où A, A', A'', A''', n, n', n'', n''', R, $R_1$ et $R_2$ ont la signification donnée dans la revendication 1, où si $R_1$ représente un hydrogène, il faut tout d'abord introduire un radical acyle $R_1'$, comme groupe protecteur, que l'on retire ensuite après la nitration par traitement avec une base.

18. Procédé de préparation du 3-(poly-)oxyalcoylamino-2'-chloro-4-nitro-4'-trifluorométhyldiphényléther de formule I, selon la revendication 1, caractérisé en ce qu'on condense un 3-amino-2'-chloro-4'-trifluorométhyldiphényléther de formule VII

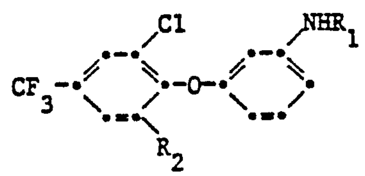

(VII)

où $R_1$ représente un hydrogène ou un alcoyle en $C_1$ à $C_4$ et $R_2$ représente un hydrogène, un chlore ou un fluor, dans un solvant organique inerte en présence d'une quantité molaire d'un agent liant d'acide, avec un halogénure de polyalcoylènoxyalcoyle de formule VIII

$$X-(A-O)_n-(A'-O)_{n'}-(A''-O)_{n''}-(A'''-O)_{n'''}-R \quad \text{(VIII)}$$

où X représente un atome d'halogène, un méthylsulfate ou un tosylate et A, A', A'', A''', n, n', n'', n''' et R ont la signification donnée dans la revendication 1 et en ce qu'on nitre ensuite avec de l'acide nitrique le produit de condensation de formule VI

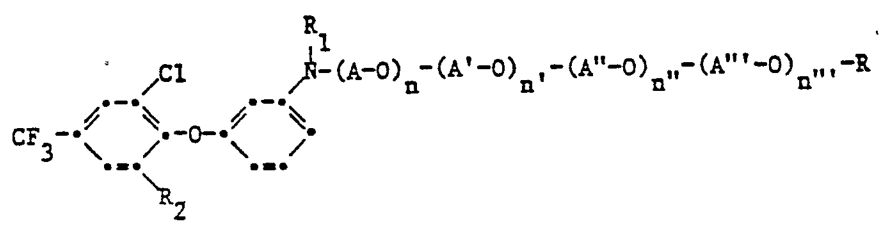

(VI)

où A, A', A'', A''', n, n', n'', n''', R, $R_1$ et $R_2$ ont la signification donnée à la revendication 1, où si $R_1$ représente un hydrogène, il faut tout d'abord introduire un radical acyle $R_1'$ comme groupe protecteur, que l'on retire ensuite à nouveau après la nitration par traitement avec une base.

19. Agent herbicide, caractérisé en ce qu'il contient outre des composants inertes, comme matière active, un 3-(poly)-oxyalcoylamino-2'-chloro-4-nitro-4'-trifluorométhyldiphényléther de formule I selon la revendication 1.

20. Application des 3-(poly-)oxyalcoylamino-2'-chloro-4-nitro-4'-trifluorométhyldiphényléthers selon la revendication 1 à la lutte sélective contre les mauvaises herbes dans les cultures de plantes utiles.

21. Application des 3-(poly-)oxyalcoylamino-2'-chloro-4-nitro-4'-trifluorométhyldiphényléthers selon la revendication 1 à la lutte sélective contre les mauvaises herbes dans les cultures de céréales, de maïs, de riz et de soja.

22. Application des 3-(poly-)oxyalcoylamino-2'-chloro-4-nitro-4'-trifluorométhyldiphényléthers selon la revendication 1 à la régulation de la croissance des plantes.

23. Procédé de lutte contre les mauvaises herbes dans les cultures de plantes utiles, caractérisé en ce qu'on traite les cultures ou leur surface cultivée avec une quantité efficace d'un 3-(poly-)-oxyalcoylamino-2'-chloro-4-nitro-4'-trifluorométhyldiphényléther selon la revendication 1.

24. Procédé de régulation de la croissance des plantes, caractérisé en ce qu'on traite les plantes ou parties de plantes dont on souhaite régler la croissance avec une quantité efficace d'un 3-(poly-)oxyalcoylamino-2'-chloro-4-nitro-4'-trifluorométhyldiphényléther selon la revendication 1.

25. Application des 3-(poly-)oxyalcoylamino-2'-chloro-4-nitro-4'-trifluorométhyldiphényléthers selon la revendication 1 à la lutte contre les insectes et les représentants de l'ordre des acariens.